Europäisches Patentamt

European Patent Office

Office européen des brevets

(11) Publication number: **0 155 515**
**B1**

(12) **EUROPEAN PATENT SPECIFICATION**

(45) Date of publication of patent specification: **20.12.89**

(51) Int. Cl.⁴: **A 61 F 13/16**

(21) Application number: **85101777.2**

(22) Date of filing: **18.02.85**

(54) Shaped napkin with elasticized edges.

(30) Priority: **21.02.84 US 581945**

(43) Date of publication of application:
**25.09.85 Bulletin 85/39**

(45) Publication of the grant of the patent:
**20.12.89 Bulletin 89/51**

(84) Designated Contracting States:
**BE CH DE FR LI LU NL**

(56) References cited:
**EP-A-0 027 303**
**EP-A-0 067 377**
**EP-A-0 091 412**
**EP-A-0 098 512**
**DE-A-2 854 792**
**DE-U-8 210 000**
**US-A-3 371 668**
**US-A-4 226 238**
**US-A-4 326 528**
**US-A-4 337 771**
**US-A-4 352 355**

(73) Proprietor: **KIMBERLY-CLARK CORPORATION**
**401 North Lake Street**
**Neenah Wisconsin 54956 (US)**

(72) Inventor: **Mokry, Patti J.**
**W4860 Blackcherry Court**
**Menasha Wisconsin (US)**

(74) Representative: **Patentanwälte Grünecker,**
**Kinkeldey, Stockmair & Partner**
**Maximilianstrasse 58**
**D-8000 München 22 (DE)**

## Description

The invention relates to a tabless elongated sanitary napkin of the type to be attached to tight fitting undergarments as defined in the preamble of claim 1.

Sanitary napkins in use suffer from two major disfunctions. One of these is leakage. In most instances leakage results not from saturation of the absorbent material, but rather fluid run off from the surface of the porous fluid permeable cover material prior to the penetration of the fluid through the cover and into the absorbent matrix. This may be due to a variety of factors, such as the nature of the fluid itself. Menses is a complex fluid which may be highly viscous and contains amongst other components cellular debris and mucoidal fraction. Both the mucoidal fraction and the cellular debris tend to collect at the surface capillaries of the cover material and block the transmission of the more aqueous component into the absorbent.

Difficulties in this downward fluid transmission are heightened by the irregular capillary size and shape and even areas of complete lack of capillaries which can occur in the manufacturing processes associated with certain nonwovens. Fluid therefore strikes the surface of the cover and is blocked or the downward transmission retarded. Fluid then tends to spread along the surface and over the side edges resulting in napkin failure even though the bulk of the absorbent layer at either end of the napkin has not even been wet. The other disfunction which has only recently been identified relate to wetness of the cover. Because tabless sanitary napkins are designed to be attached to tight fitting undergarments, the surface of the nonwoven cover material is in direct contact with the perineal area, because of the tight fitting undergarments used by the wearer. Since the nonwoven cover materials are inherently nonwettable, there is a wet or damp surface feeling associated with them even when there is adequate downward transmission.

Attempts have been made to increase comfort of tabless sanitary napkins as well as to ensure greater surface contact in the perineal area by providing napkins with inwardly arcuate sides. These napkins supposably conform to the shape of the thighs adjacent the perineal area and therefore will suffer less distortion from movement by the wearer and it is theorized provide better fluid transmission downward into the absorbent component and due to the better surface interface between the perineal area and the cover.

A sanitary napkin of the type having the features of the preamble of claim 1 is disclosed in EP—A—0 091 412. The known sanitary napkin comprises elastic means attached to straight lateral sides of the napkin causing the sides to be raised and to form side flaps. Fluid will be collected between the side flaps and the central area, with the side flaps forming barriers which should prevent lateral leakage of fluid.

In another type of sanitary garment, i.e. diapers, there is generally not the intimate contact between the crotch of the wearer and the containment garment. Diapers are, of course, of a vastly different configuration. Diapers, even though they include a fluid pervious non-woven cover, an absorbent and a fluid impermeable baffle, are designed to be an undergarment with complete encircling of the legs and waist. Side leakage is prevented in diapers by the utilisation of elastic completely around the leg area to provide a seal. US—A—4 326 528 discloses a diaper which is arcuate in cross-sectional profile and constructed to form an containment pocket. US—A—4 226 238 describes a rather complicated diaper construction in which a slightly elasticated central pouch is formed. US—A—3 371 668 describes a sanitary napkin which utilises elastic for certain constructional purposes. This type of seal is impossible to be obtained with a sanitary napkin which is not designed to be a garment with encircling means around the leg opening. DE—A—28 54 792 discloses an absorbent pad which may be used as a sanitary napkin. The elastic means of this pad extend over more than the half of the entire length and is said to ensure a tight fit to the legs of the wearer also. Thus, this publication also discloses a napkin which is used as an undergarment.

Thus, it is an object of the present invention to increase the comfort of a tabless sanitary napkin of the type to be attached to tight fitting undergarments.

In accordance with the invention, the napkin is provided with inwardly arcuate sides, with the elastic means positioned in the arcuate sides and the absorbent layer forms a cup-shaped profile, with the central portion of the napkin forming the deepest part of the cup, allowing the cup to act as a reservoir.

The napkin of the present invention is constrained by the elastic in relaxed state to form a cup-shaped configuration with the central portion of the napkin forming the deepest part of the cup. This "bottom of the cup" corresponds generally to the perineal area and therefore the possibilities of direct contact between the perineal area and the cover are minimized particularly when the wearer is in a standing or reclining position. The configuration of the napkin allows the central portion to act as a reservoir with additional time for the fluid to penetrate the cover. Due to the presence of the elastic at the sides and the fact that the central portion receiving the fluid is lower than the sides, side leakage is virtually impossible also.

Also because of the minimal amount of contact between the body of the wearer and the surface of the cover, the undesirable wet feeling associated with the intimate contact is avoided.

Preferred embodiments of the invention are described in claims 2 to 5.

The invention may be more readily understood by reference to one embodiment shown in the drawings in which:

Fig. 1 is a plan view of the napkin and cross-section;

Fig. 2 is a side perspective view of a napkin which is partially turned toward the viewer;

Fig. 3 is a side perspective view of the napkin according to this invention.

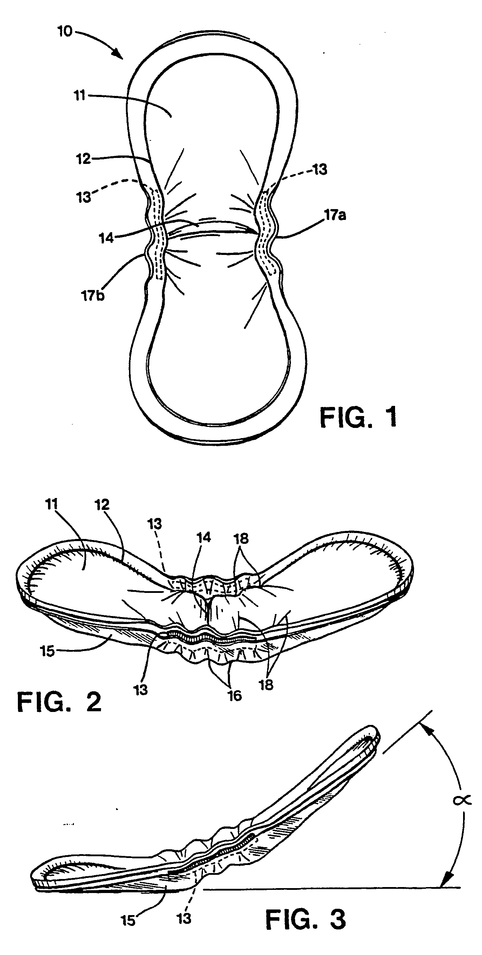

As can be seen by reference to Fig. 1 the napkin 10 of this invention has generally inwardly arcuate sides 17a and 17b. The cover 11 is sealed inward from the outer edge of the napkin by seal line 12. This seal may be either by adhesive means or preferably a continuous or discontinuous ultrasonic band. Elastic elements 13 are added at the approximate center of each longitudinal side edge of a napkin outward of the seal line 12.

The napkin is essentially planar when the elastic is applied in a stretched condition and attached, e.g. by adhesive means. The elastic strips 13, when returning to their relaxed position, distort the planar configuration of the napkin by pulling the ends of the napkin upward toward each other and the sides of the napkin inward. This distortion forms the cup-like profile 14.

One particularly preferred method of introducing elastic is by utilizing an extrudable elastic which is added initially as a liquid and which upon cooling both adheres and becomes elastic. This eliminates the separate step of adhesively bonding strips of elastic to either side. An example of such a product is described in U.S. Patent 4,259,220 assigned to H. B. Fuller Company in Saint Paul, Minnesota.

The general configuration of this napkin can more readily be seen by reference to Fig. 2 in which the baffle 15 is shown providing the outside surface of the cup and the cover 11 the inside surface with the bottom portion of the cup 14 shown as a small fold. There are also constriction 16 shown along the central portion of the baffle 15 with corresponding constrictions 18 present along the central portion edge of the cover 11. The extent of the folds 16, 14 and 18 are dependent upon the width and length of the elastic, the stiffness of the absorbent layer (not shown) and the napkin in a whole as well as the degree of elasticity of the elastic bands 13. These factors are balanced, so that, as shown in Fig. 3, the angle formed by the outer profile of the edge of the napkin with the plane upon which the napkin rests is upon 30° and 90°. The benefits associated with this napkin when the angle is less than 30° essentially disappear and it is extremely difficult when the angle is greater than 90° to maintain the proper napkin configuration during use.

For purposes of wear comfort is desired that the elastic width be controlled between 1/16 and 5/16 of an inch (1.6 mm and 8 mm) and currently preferred as a width of 3/16" (5 mm). Bands much beyond 5/16" (8 mm) tend to chafe and become uncomfortable for the wearer. It is also desirable that the elastic be inset at least 1/16" (1.6 mm) from the side edges. If a self-adhering elastic is not employed, it may be necessary to provide another seal closer to the edge of the napkin although this seal need not be continuous. The reason for the second seal would be to minimize the exposure to the edges of the baffle and the cover which also could provide chafing. It is generally been found that it is necessary only to extend the elastic from 10—30% of the length of the side edges of the napkin, of course, this will vary depending upon the degree of elasticity, the stiffness and flexibility of the napkin, and other factors discussed above.

It has been found that the best configuration results from placing the elastic outside of an initial seal line, although some of the benefits of this invention may be obtained by placing the elastic exactly at or slightly inside of the seal line.

## Claims

1. A tableless elongated sanitary napkin (10) of the type to be attached to tight fitting undergarments including a fluid impermeable baffle (15), a fluid pervious cover (11), the baffle (15) and the cover (11) being sealed at the sides of the napkin (10), an absorbent layer positioned between the cover (11) and the baffle (15), and elastic means (13) centrally positioned at the sides of the napkin (10), characterized in that the napkin (10) is formed with inwardly arcuate sides (17a, 17b) with the elastic means (13) positioned in the arcuate sides (17a, 17b) and forming the absorbent layer to a cup-shaped profile (14) with the central portion of the napkin (10) forming the deepest part of the cup (14) allowing the cup (14) to act as a reservoir.

2. The napkin according to claim 1 wherein the elastic means (13) extends between 10 and 30% of the length of the sides.

3. The napkin according to claim 1 or 2 wherein the angle (2) formed by a line tangent to the outer portion of the side of the baffle (15) of the napkin (10) in profile and a plane extending along the bottom of the napkin (10) is between 30° and 90°.

4. The napkin according to any of claims 1 to 3 wherein the width of the elastic means (13) is between 1/16" (1.6 mm) and 5/16" (8 mm).

5. The napkin according to any of claims 1 to 4 wherein the elastic means (13) should be inset at least 1/16" (1.6 mm) from the side layer.

## Patentansprüche

1. Eine langgestreckte Sanitärbinde (10) ohne Befestigungsbänder des in dichter Anpassung in der Unterwäsche anzuordnenden Typs, mit einer flüssigkeitsundurchlässigen Sperrschicht (15), einer flüssigkeitsdurchlässigen Deckschicht (11), wobei die Sperrschicht (15) und die Deckschicht (11) an den Seiten der Binde (10) miteinander verbunden sind, mit einer zwischen der Deckschicht (11) und der Sperrschicht (15) angeordneten absorbierenden Schicht, und mit einer an den Seiten der Binde (10) zentral angeordneten elastischen Einrichtung (13), dadurch gekennzeichnet, daß die Binde (10) mit einwärts gebogenen Seiten (17a, 17b) versehen ist, wobei die elastische Einrichtung (13) an den gebogenen Seiten (17a, 17b) angeordnet ist und die absorbierende Schicht in ein schalenförmiges Profil (14) formt, bei dem der zentrale Bereich der Binde (10) den tiefsten Teil der Schale (14) bildet, wodurch die Schale (14) als Reservoir dienen kann.

2. Binde nach Anspruch 1, bei der die elastische Einrichtung (13) sich zwischen 10 und 30% der Länge der Seiten erstreckt.

3. Binde nach Anspruch 1 oder 2, bei der der Winkel (2), der durch eine tangential zum äußeren Bereich der Sperrschicht-Seite (15) der Binde (10) im Profil verlaufende Linie und eine sich entlang dem Boden der Binde (10) erstreckende Ebene gebildet ist, zwischen 30° und 90° beträgt.

4. Binde nach einem der Ansprüche 1 bis 3, bei der die Breite der elastischen Einrichtung (13) zwischen 1/16″ (1,6 mm) und 5/16″ (8 mm) liegt.

5. Binde nach einem der Ansprüche 1 bis 4, bei der die elastische Einrichtung (13) mindestens 1/16″ (1,6 mm) von der Seitenschicht eingesetzt sein sollte.

**Revendications**

1. Serviette hygiénique sans attache de forme allongée (10) du type devant être fixé à des sous-vêtements près du corps comprenant un film imperméable au flux menstruel (15), une couverture perméable au flux menstruel (11), le film (15) et la couverture (11) étant scellés sur les côtés de la serviette (10), une couche absorbante disposée entre la couverture (11) et le film (15), et un moyen élastique (13) disposé de manière centrale sur les côtés de la serviette (10), caractérisée en ce que la serviette (10) est formée de manière à ce que les côtés (17a, 17b) soient courbés vers l'intérieur, le moyen élastique (13) disposé dans les côtés courbés (17a, 17b) et faisant prendre à la couche absorbante un profil en forme de coupe (14), la partie centrale de la serviette (10) formant la partie la plus profonde de la coupe (14) et permettant à la coupe (14) de servir de réservoir.

2. Serviette selon la revendication 1, dans laquelle le moyen élastique (13) s'étend sur 10 à 30% de la longueur des côtés.

3. Serviette selon la revendication 1 ou 2, dans laquelle l'angle (2) formé par une ligne tangente à la partie extérieure du côté du film (15) de la serviette (10) de profil et un plan s'étendant le long du fond de la serviette (10) va de 30 à 90 degrés.

4. Serviette selon l'une des revendications 1 à 3, dans laquelle la largeur du moyen élastique (13) va de 1/16 de pouce (1,6 mm) à 5/16 de pouce (8 mm).

5. Serviette selon l'une des revendications 1 à 4, dans laquelle le moyen élastique (13) doit être en retrait d'au moins 1/16 de pouce (1,6 mm) par rapport à la couche latérale.

FIG. 1

FIG. 2

FIG. 3